# EUROPEAN PATENT APPLICATION

(11) **EP 1 982 639 A2**
(43) Date of publication of application: **22.10.2008**
(21) Application number: 08006825.7
(22) Date of filing: 03.04.2008
(51) Int. Cl.: A61B 1/05, H04N 5/232, H04N 7/18

(54) **Electronic endoscope apparatus**

(30) Priority: 20.04.2007 JP 2007112123
(71) Applicant: Olympus Medical Systems Corp., Tokyo 151-0072 (JP)
(72) Inventor: Niida, Koichi, Shibuya-ku Tokyo 151-0072 (JP); Honda, Takemitsu, Shibuya-ku Tokyo 151-0072 (JP); Yamashita, Shinji, Shibuya-ku Tokyo 151-0072 (JP); Saito, Katsuyuki, Shibuya-ku Tokyo 151-0072 (JP)
(74) Representative: von Hellfeld, Axel

(57) **Abstract**

An electronic endoscope apparatus includes: an electronic endoscope (1) having a power amplifier (17) for transmitting by radio or by cable an image pickup signal obtained from an image sensor (11); and a low noise amplifier (22) for receiving the image pickup signal transmitted by radio and by cable. The apparatus further includes: a processor unit (2) for producing a video signal from the image pickup signal; and a cable connection detection circuit (18) for changing gain of the power amplifier (17) or gain of the low noise amplifier (22), upon detecting a connection of a cable between the electronic endoscope (1) and the processor unit (2), for transmitting the image pickup signal by cable.

## Description

### BACKGROUND OF THE INVENTION

### 1. Field of the Invention

The present invention relates to an electronic endoscope apparatus, and in particular, to an electronic endoscope apparatus which is able to change gain.

### 2. Description of the Related Art

Generally, conventional electronic endoscope apparatuses is configured to include an electronic endoscope and a processor unit. The electronic endoscope transmits an image pickup signal of a subject to be observed to the processor unit via a cable, the subject being picked up by an image sensor provided in the electronic endoscope. The processor unit performs image processing of the image pickup signal and outputs a video signal generated by the image processing to a monitor or the like.

Japanese Patent Laid-Open No. 2001-45472 discloses an endoscope apparatus in which an image pickup signal obtained by an endoscope is adapted to be transmitted to a processor unit by radio rather than by cable. However, radio transmission alone may possibly distort the image pickup signal to be transmitted, because of a possible location of an obstacle in the transmission path, for example. Thus, in order to avoid the distortion of an image pickup signal by radio transmission, it is considered to perform also by cable transmission. This, however, it is necessary to provide another transmission circuit for the cable transmission in addition to the transmission circuit for the radio transmission. Providing different transmission circuits for cable transmission and radio transmission, however, may cause to increase the circuit size, thereby increasing a size of the endoscope apparatus. In addition, different transmission circuits for cable transmission and radio transmission needs switching means for switching the transmission circuits according to a state of use in which signal transmission is performed by radio or by cable, thereby leading to a complicated switching operation.

Japanese Patent Laid-Open No. 2004-159833 discloses an electronic endoscope having an integrated transmission circuit for cable and radio transmissions. This electronic endoscope is configured to enable transmission of an image pickup signal modulated for radio transmission, by cable or radio transmission. The electronic endoscope apparatus disclosed in this Japanese Patent Laid-Open No. 2004-159833 enables transmission of an image pickup signal using a single transmission circuit when transmission is performed both by cable and by radio.

However, an electronic endoscope apparatus using a single transmission circuit for cable and radio transmissions cannot optimize transmission gain between cable and radio transmissions, leading to possible occurrences of transmission failure. Thus, the electronic endoscope disclosed in Japanese Patent Laid-Open No. 2004-159833 has raised a problem that reliable transmission quality is not ensured.

Here, an object of the present invention is to provide an electronic endoscope apparatus which eliminates the complicated switching operation. Specifically, the present invention is purposed to provide an electronic endoscope apparatus which does not need to include different transmission circuits for cable transmission and radio transmission, can reduce a size of the electronic endoscope apparatus, and also can switch between cable transmission and radio transmission by attaching/detaching a cable. Further, the present invention also has an object of providing an electronic endoscope apparatus which causes no transmission failure in cable and radio transmissions, by optimizing transmission gain between cable transmission and radio transmission.

### SUMMARY OF THE INVENTION

An electronic endoscope of the present invention comprises: an electronic endoscope including a transmission circuit for performing radio transmission and cable transmission of an image pickup signal obtained by an image pickup element; a processor unit including a reception circuit for receiving the image pickup signal transmitted by radio transmission and cable transmission, and for producing a video signal from the image pickup signal; and gain changing means for changing gain of the transmission circuit and gain of the reception circuit, upon detecting connection of a cable between the electronic endoscope and the processor unit, the connection being for performing cable transmission of the image pickup signal.

According to the electronic endoscope apparatus of the present invention, it is not necessary to provide different transmission circuits for cable transmission and radio transmission, whereby the size of the apparatus can be reduced. Also, the electronic endoscope apparatus of the present invention is able to perform switching between cable transmission and radio transmission by attaching/detaching a cable, whereby a complicated switching operation can be eliminated. Further, the electronic endoscope apparatus of the present invention is able to optimize transmission gain between cable transmission and radio transmission, whereby the occurrences of transmission failure in cable and radio transmissions can be reduced.

### BRIEF DESCRIPTION OF THE DRAWINGS

Fig. 1 is a block diagram illustrating a configuration of an electronic endoscope apparatus according to a first embodiment of the present invention;
Fig. 2 is a block diagram illustrating a configuration of an electronic endoscope apparatus obtained by arranging a switching unit in the first embodiment;
Fig. 3 is a block diagram illustrating a configuration of an electronic endoscope apparatus according to a second embodiment of the present invention;
Fig. 4 is a circuit diagram illustrating a configuration of an attenuator 26 shown in Fig. 3;
Fig. 5 is a block diagram illustrating a configuration of an electronic endoscope apparatus obtained by arranging a switching unit in the second embodiment; and
Fig. 6 is a block diagram illustrating a configuration for reducing power consumption of a processor unit.

### DETAILED DESCRIPTION OF THE PREFERRED EMBODIMENTS

With reference to the drawings, hereinafter will be described some embodiments of the present invention.

### <First Embodiment>

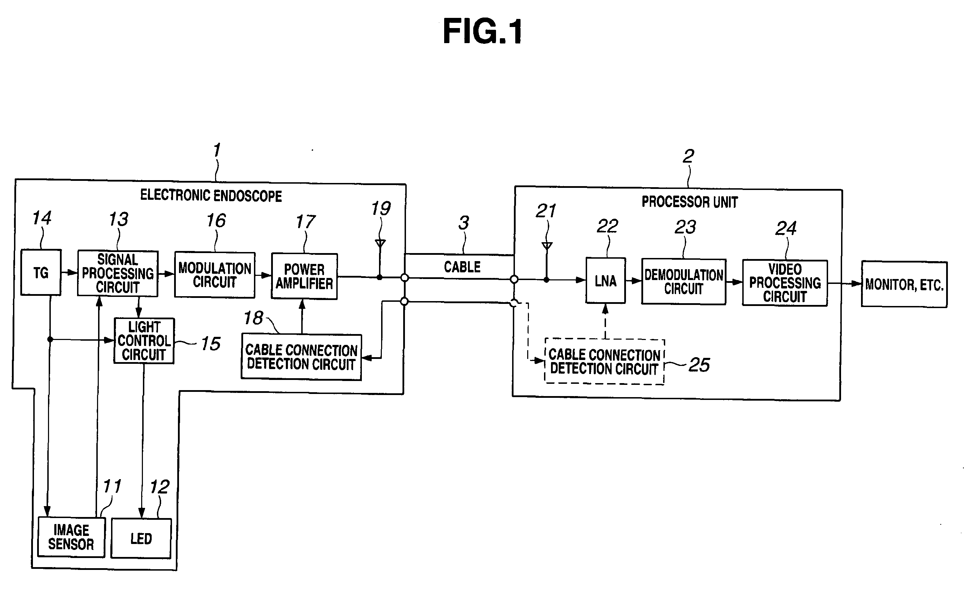

Fig. 1 is a block diagram illustrating a configuration of an electronic endoscope apparatus according to the present embodiment.

The electronic endoscope apparatus includes an electronic endoscope 1 having an insertion portion, a processor unit 2, and a detachable cable 3 which is detachable with respect to the electronic endoscope 1 and the processor unit 2. The electronic endoscope 1 includes an image sensor 11, a light emitting diode (hereinafter referred to as an "LED") 12, a signal processing circuit 13, a timing generator (hereinafter referred to as a "TG") 14, a light control circuit 15, a modulation circuit 16, a power amplifier 17, a cable connection detection circuit 18, and a transmission antenna 19.

The processor unit 2 includes a reception antenna 21, a low noise amplifier (hereinafter referred to as an "LNA") 22, a demodulation circuit 23, and a video processing circuit 24. The cable 3 is a coaxial cable having a signal line and a grounding line. The cable 3 is configured to be detachable with respect to the electronic endoscope 1 and the processor unit 2. As an alternative to this, one end of the cable 3 may be fixed to the side of the electronic endoscope.

The insertion portion, which is inserted into a body cavity of a patient, is provided with the image sensor 11 and the LED 12 at its distal end. The image sensor 11 converts an image of the subject, which is illuminated by the light of the LED 12, into an image pickup signal and outputs the converted signal to the signal processing circuit 13. The signal processing circuit 13 applies sampling, clamping, analog-to-digital (hereinafter referred to as "AD") conversion and data coding to the inputted image pickup signal and outputs the resultant signal to the modulation circuit 16. Also, the signal processing circuit 13 can superimpose, as required, an ON/OFF signal of a switch, not shown, provided at the electronic endoscope and a signal indicative of an individual number of the electronic endoscope, over the video signal.

The outputted signal of the signal processing circuit 13 is outputted to the light control circuit 15 as well as the modulation circuit 16. The light control circuit 15 detects the outputted signal, that is video signal, of the signal processing circuit 13 and controls lighting time of the LED 12 using a pulse width modulation (hereinafter referred to as "PWM") scheme, so that the luminance of an image have a predetermined value, or that the LED 12 light the subject with a predetermined brightness. Accordingly, the light control circuit 15 includes a drive circuit, not shown, for the LED 12.

The TG 14 outputs a suitable timing signal to each of the image sensor 11, the signal processing circuit 13 and the light control circuit 15. A timing signal for driving is inputted to the image sensor 11. A timing signal for signal processing is inputted to the signal processing circuit 13. A timing signal for light control is inputted to the light control circuit 15. The TG 14 includes a drive circuit, not shown, for the image sensor 11.

The modulation circuit 16 modulates the image pickup signal outputted from the signal processing circuit 13 into a carrier signal, which is then outputted to the power amplifier 17. The cable connection detection circuit 18 detects connection of the cable 3 and outputs a detection signal indicative of the connection of the cable to the power amplifier 17 as connection information on the cable 3. The power amplifier 17 serving as a transmission circuit amplifies the carrier signal outputted from the modulation circuit 16 with voltage gain for radio transmission or voltage gain for cable transmission, on the basis of the output of the cable connection detection circuit 18, and outputs the amplified signal to the transmission antenna 19 or the cable 3. Thus, the cable connection detection circuit 18 constitutes cable connection detecting means, or a cable connection detecting unit, and gain changing means, or a gain changing unit. In radio transmission, the carrier signal is transmitted to the processor unit 2 via the transmission antenna 19. On the other hand, in cable transmission, the carrier signal is transmitted to the processor unit 2 via the cable 3.

The carrier signal transmitted by radio is received by the reception antenna 21 and inputted to the LNA 22. The carrier signal transmitted by cable is passed through the cable 3 and inputted to the LNA 22. The LNA 22 serving as a reception circuit amplifies the inputted carrier signal and outputs the amplified signal to the demodulation circuit 23. The demodulation circuit 23 demodulates the carrier signal outputted from the LNA 22 into an image pickup signal, for output to the video processing circuit 24. The video processing circuit 24 has functions of gamma correction processing, color tone adjustment processing, insulation processing for ensuring electrical safety for the inputted image pickup signal in performing cable connection, and superposition processing for characters such as letters. Thus, the inputted image pickup signal is converted by the video processing circuit 24 into a video signal corresponding to an external display device, such as a monitor. The video signal resulting from the conversion performed by the video processing circuit 24 is outputted to a monitor or the like so that a subject image can be displayed on the monitor or the like.

Next, an operation of the embodiment configured as described above is described.

First, an operation in radio transmission without the connection of the cable 3 will be described. The signal processing circuit 13 applies a processing of sampling or the like to the image pickup signal derived from the image sensor 11 and outputs the processed signal to the modulation circuit 16. The modulation circuit 16 modulates the processed signal and outputs the modulated carrier signal to the power amplifier 17.

In this case, since the cable 3 is not connected, the cable connection detection circuit 18 outputs a gain switching signal to the power amplifier 17 that serves as a transmission circuit to give instructions for gain switching thereto so that radio transmission can be performed. Based on the gain switching signal, the power amplifier 17 amplifies the inputted carrier signal with the voltage gain for radio transmission, and outputs the carrier signal that has been amplified with the voltage gain for radio transmission, to the transmission antenna 19.

The carrier signal that has been radio-transmitted from the transmission antenna 19 is inputted to the demodulation circuit 23 via the LNA 22 that serves as a reception circuit. The demodulation circuit 23 demodulates the carrier signal outputted from the LNA 22 into an image pickup signal, to output to the video processing circuit 24. The video processing circuit 24 applies gamma correction processing and the like to the inputted image pickup signal, and outputs the image pickup signal to a monitor or the like, so that the subject image can be observed.

Next, an operation in cable transmission with the connection of the cable 3 is described. Upon detecting connection with the cable 3, the cable connection detection circuit 18 outputs a detection signal to the power amplifier 17. The detection signal, or gain switching signal, gives instructions for gain switching to the power amplifier 17 so that cable transmission can be performed. Based on the gain switching signal, the power amplifier 17 amplifies the inputted carrier signal with the voltage gain for cable transmission, and outputs the carrier signal that has been amplified with the voltage gain for cable transmission, to the cable 3.

The carrier signal transmitted via the cable 3 is inputted to the LNA 22. The LNA 22 applies low noise amplification processing to the inputted carrier signal and outputs the amplified carrier signal to the demodulation circuit 23. The demodulation circuit 23 then demodulates the carrier signal outputted from the LNA 22 into an image pickup signal, to output to the video processing circuit 24. The video processing circuit 24 applies gamma correction processing and the like to the inputted image pickup signal and outputs a video signal to a monitor or the like, so that a subject image can be observed.

In this case, a setting value at the power amplifier 17 in the cable transmission is set so that the amplitude of the carrier signal inputted to the LNA 22 in cable transmission is equal to the amplitude of the carrier signal inputted to the LNA 22 in radio transmission. The propagation loss of the carrier signal is smaller in cable transmission in which the carrier signal propagates through a conductive material, than that in radio transmission in which the carrier signal propagates through the air. Accordingly, the setting value of gain at the power amplifier 17 in cable transmission can be made smaller than the setting value of gain at the power amplifier 17 in radio transmission. Thus, the amplitude of the carrier signal inputted to the LNA 22 in cable transmission can be optimized so as to be substantially constant in cable and radio transmissions, rather than transmitting the carrier signal amplified for radio transmission as it is in cable transmission. With this arrangement, reception failure can be reduced, the failure having been caused by an excessively large carrier signal inputted into the LNA 22 for saturation of the carrier signal, as in the conventional cases.

In cable transmission, even when the carrier signal that has been set at the gain for cable transmission is transmitted from the transmission antenna 19, such a carrier signal is insufficient for a carrier signal to be used in radio transmission, since the gain which is sufficient for radio transmission has not been set at the power amplifier 17. Thus, no influence is given on the observation of the subject image in the cable transmission. Other operations are the same as in radio transmission, and thus the subject image can be observed in the similar manner to that in radio transmission.

Thus, the present embodiment can realize the electronic endoscope apparatus which does not need to provide different transmission circuits for cable and radio transmissions, whereby the size of the apparatus can be reduced. Also, the apparatus according to the present embodiment enables switching between cable and radio transmissions by attaching/detaching a cable, and thus can eliminate the complicated switching operation. Further, the present embodiment can realize the electronic endoscope apparatus which can optimize transmission gain between cable and radio transmissions, whereby the occurrences of transmission failure in cable and radio transmissions can be reduced.

Furthermore, the electronic endoscope apparatus according to the present embodiment can reduce power consumption since the setting value of gain at the power amplifier 17 is small in cable transmission. Accordingly, when a battery is used as a power source, not shown, of the electronic endoscope 1, the usable time period of the electronic endoscope 1 can be extended. Also, due to the small amplitude of the carrier signal transmitted via the cable 3, unnecessary emission of noise in the air from the coaxial cable can be advantageously reduced. Similarly, advantageous reduction can also be achieved in the unnecessary emission of noise in the air from the connecting portions between the coaxial cable and the electronic endoscope 1, and between the coaxial cable and the processor unit 2.

As shown in Fig. 1, a cable connection detection circuit 25 may be arranged in the processor unit 2, instead of arranging the cable connection detection circuit 18 in the electronic endoscope 1. Thus, the setting value of gain at the LNA 22 at the time of receiving signals can be switched on the basis of the gain switching signal outputted from the cable connection detection circuit 25. In this case, since the gain for radio transmission is set at the power amplifier 17, an excessively large carrier signal that has been amplified with the gain for radio transmission is transmitted via the cable 3. Thus, although no advantageous reduction can be expected in the unnecessary emission of noise as in the case where the cable connection detection circuit is arranged in the electronic endoscope 1, this arrangement can reduce the circuit size of the electronic endoscope 1.

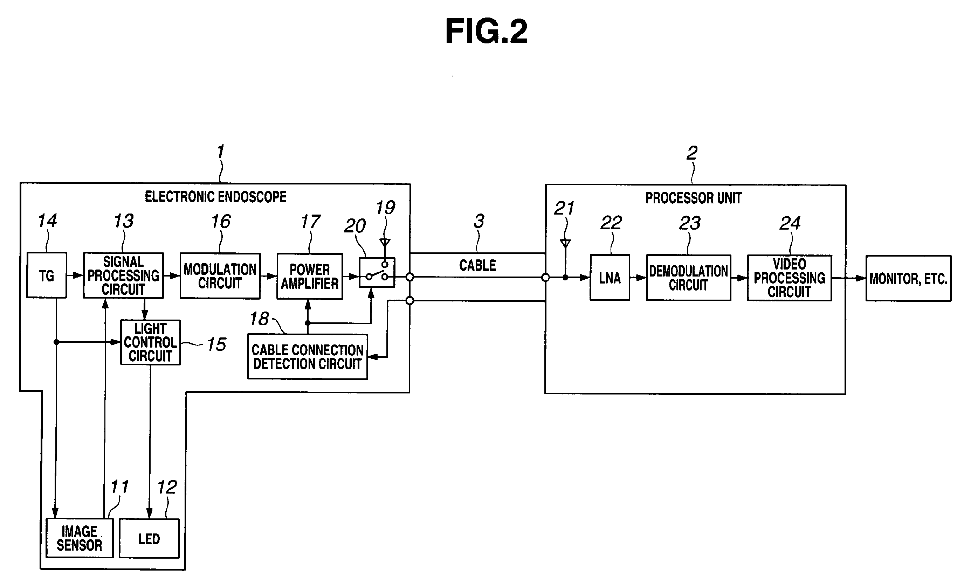

Further, as shown in Fig. 2, a switching unit, or switching means 20, may be arranged, so that the connection of the output of the power amplifier 17 in the electronic endoscope 1 can be switched between the antenna 19 and the cable 3. Fig. 2 is a block diagram illustrating a configuration of the electronic endoscope apparatus provided with the switching unit. The electronic endoscope 1 can selectively switch between radio and cable transmissions for the transmission of the carrier signal outputted from the power amplifier 17, on the basis of the gain switching signal outputted from the cable connection detection circuit 18. Such a configuration can prevent noise from being generated in cable transmission by the unnecessary emission of the noise from the antenna.

In addition, the shapes of the connectors for the antenna and the cable may be made identical so as to provide a structure which does not allow connection of the cable 3 without removing the antenna, when the cable is connected. Similarly, noise can be prevented from being generated in cable transmission by the unnecessary emission of the noise from the antenna.

### <Second Embodiment>

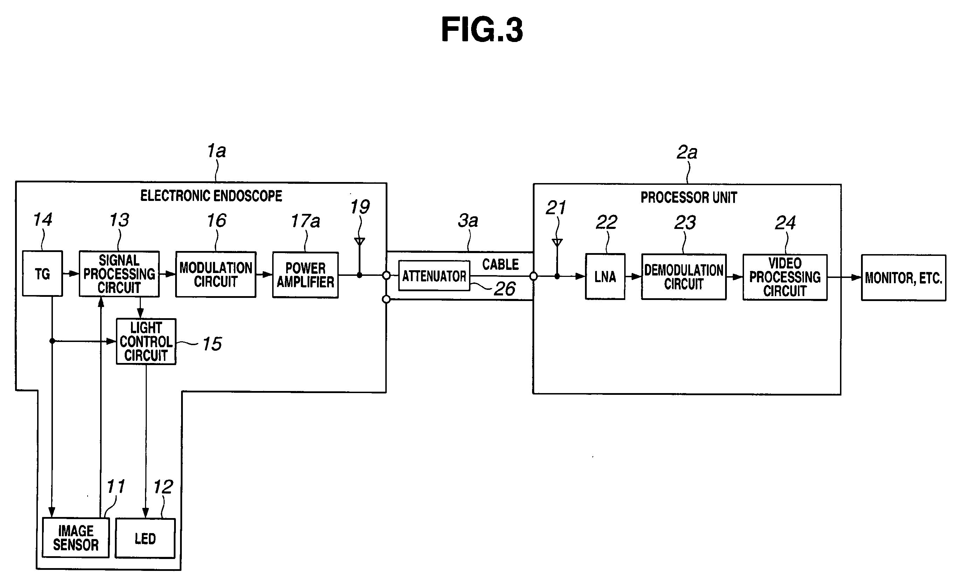

Fig. 3 is a block diagram illustrating a configuration of an electronic endoscope apparatus according to a second embodiment. In Fig. 3, the components identical with those in Fig. 1 are designated with the same references for the omission of explanation.

As shown in Fig. 3, in the electronic endoscope apparatus according to the present embodiment, an electronic endoscope 1a is not provided with a cable connection detection circuit. Instead, an attenuator 26 is provided at a midway of a cable 3a. Other structures are configured in the same manner as that in the first embodiment.

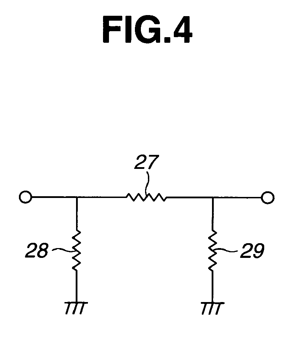

Fig. 4 is a circuit diagram illustrating a configuration of the attenuator 26. As shown in Fig. 4, the attenuator 26 is structured by a circuit using resistors 27 to 29. The resistor 27 is disposed at the midway of a signal line of the cable 3a. Each of the resistors 28 and 29 is connected at the midway of each of respective ground lines laterally provided at the resistor 27. This configuration does not require the supply of a power source for the attenuator 26 at the cable 3a, whereby the attenuator 26 can be structured by only the signal line and the ground lines. The attenuator 26 may be structured by a circuit using a semiconductor.

Since no cable connection detection circuit is provided in the electronic endoscope 1a, a power amplifier 17a cannot switch gains. Therefore, a carrier signal inputted to the power amplifier 17a is amplified by the voltage gain for radio transmission and outputted to the transmission antenna 19 or the cable 3a. In cable transmission, the carrier signal outputted from the power amplifier 17a is attenuated in its amplitude by the attenuator 26 and transmitted to the LNA 22 in a processor unit 2a. Configuration of other portions and configuration in radio transmission are the same as in the first embodiment. Thus, a subject image is displayed in a monitor or the like in the same manner as that in the first embodiment.

Next, an operation of the present embodiment configured described above will be described. The description on the same operations as in the first embodiment is omitted.

First, an operation in radio transmission without the connection of the cable 3a is described. Since no cable connection detection circuit is provided in the electronic endoscope 1a, gain for radio transmission is set at the power amplifier 17a. The power amplifier 17a amplifies an inputted carrier signal with the voltage gain for radio transmission and outputs the amplified carrier signal to the transmission antenna 19. Other operations are the same as that in the first embodiment, and thus a subject image can be observed in the similar manner.

An operation in cable transmission with the connection of the cable 3a is described. Since the voltage gain for radio transmission is set at the power amplifier 17a, the inputted carrier signal is amplified with the voltage gain for radio transmission and the amplified carrier signal is outputted to the cable 3a. The carrier signal inputted to the cable 3a is attenuated in its amplitude by the attenuator 26 and outputted to the LNA 22 of the processor unit 2a. In other words, the attenuator 26 constitutes a correction part, or correcting means, for correcting the amplitude of the carrier signal.

In this case, the resistances of the resistors 27 to 29 of the attenuator 26 are set so that the amplitude of the carrier signal inputted to the LNA 22 in cable transmission is equal to the amplitude of the carrier signal inputted to the LNA 22 in radio transmission. When a carrier signal amplified for radio transmission is transmitted by cable, the excessively large carrier signal amplified for radio transmission is attenuated by the attenuator 26, whereby the amplitude of the carrier signal inputted to the LNA 22 can be optimized. In this way, reception failure can be reduced, the failure having been conventionally caused by an excessively large carrier signal inputted into the LNA 22 for saturation of the carrier signal.

Thus, the present embodiment can realize the electronic endoscope apparatus which does not need to provide different transmission circuits for cable and radio transmissions, whereby the size of the apparatus can be reduced. Also, the apparatus according to the present embodiment enables switching between cable and radio transmissions by attaching/detaching a cable, and thus can eliminate the complicated switching operation. Further, the present embodiment can realize the electronic endoscope apparatus which can optimize transmission gain between cable and radio transmissions, whereby the occurrences of transmission failure in cable and radio transmissions can be reduced.

Further, by arranging the attenuator 26 in the vicinity of the electronic endoscope 1a, the unnecessary emission of noise into the air from the coaxial cable can be reduced. To be more specific, the attenuator 26 is arranged in the vicinity of a connector which connects the coaxial cable to the electronic endoscope 1a.

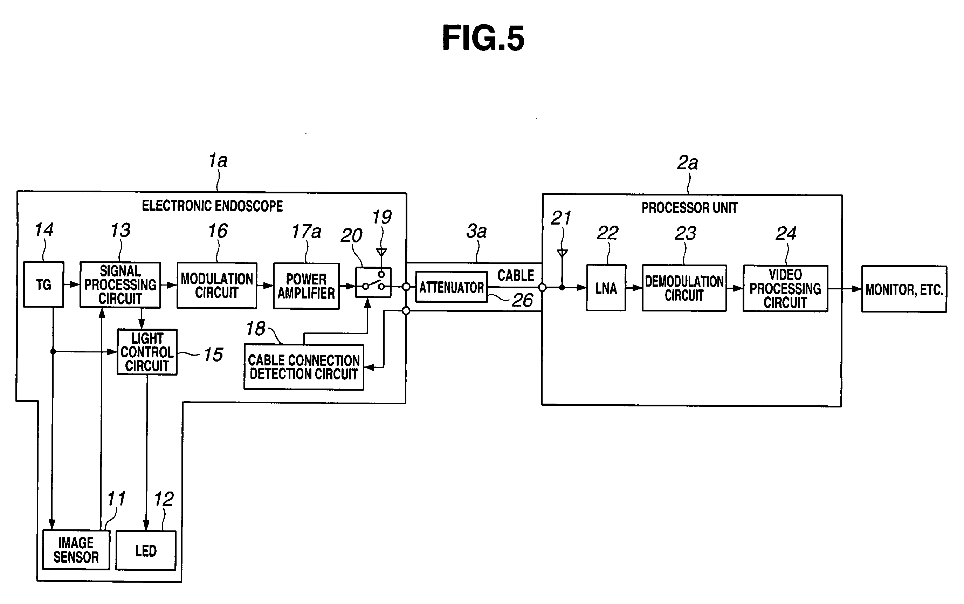

As shown in Fig. 5, the switching means 20 may be provided in the electronic endoscope 1a so as to perform switching between the antenna 19 and the cable 3 a for connection with the output of the cable connection detection circuit 18 and the output of the power amplifier 17a. The electronic endoscope 1a can perform selective switching between radio transmission and cable transmission for the carrier signal outputted from the power amplifier 17a, on the basis of the gain switching signal outputted from the cable connection detection circuit 18. Such a configuration can prevent noise from being generated in cable transmission by the unnecessary emission of the noise from the antenna.

In addition also in the present embodiment, the shapes of the connectors for the antenna and the cable may be made identical so as to provide a structure which does not allow connection of the cable 3 without removing the antenna, when need arises to establish connection with the cable. In this case as well, noise can be prevented from being generated in cable transmission by the unnecessary emission of the noise from the antenna.

In the first and second embodiments which use a battery as a power supply unit, or power supplying means, for the electronic endoscope, an important issue is reducing power consumption in order to enable use of the electronic endoscope over a long period of time.

In order to solve this issue, controlling a lighting period of an LED using the PWM control scheme mentioned above or a current controlling scheme for controlling the amount of current supplied to the LED, can reduce more power consumption than that of constantly lighting the LED to pick up images using an electronic shutter of the image sensor 11.

In the case where the outputted signal of the image sensor 11 does not substantially vary and where the results of the detection for controlling the light of the LED do not vary for a certain period of time, the electronic endoscope is detected as being in a state out of use, i.e. a standby state, before or after examination. In this regard, when the electronic endoscope is detected as being in the standby state before or after examination, the electronic endoscope may be adapted to transfer to a low current consumption mode so as to reduce the lighting time of the LED or to reduce current consumption, which is led to the reduction of the power consumption.

In cable transmission, the electronic endoscope and the processor unit are necessarily connected through the cable. Therefore, the cable may be provided with a power supply line and the processor unit may be provided with a power supply unit, or power supplying means, so that, even when the remaining amount of the battery becomes small, electrical power can be supplied to the electronic endoscope via the cable and the electronic endoscope can be used over a prolonged period of time. Alternatively, the electrical power to be supplied to the electronic endoscope may be supplied to a chargeable battery, so that the battery can be charged. Since this may allow the electrical power to be supplied from the processor unit in cable transmission, lighting level of the LED as well as gain can be increased to give priority to the brightness of the endoscopic image without restriction on power consumption.

Different operation modes, that is, a cable transmission mode for placing priority on brightness and a radio transmission mode for placing priority on power consumption reduction, may be provided to the electronic endoscope. Thus, according to the state of use of the electronic endoscope in radio and cable transmissions, the operation modes may be switched to improve usability of the electronic endoscope apparatus.

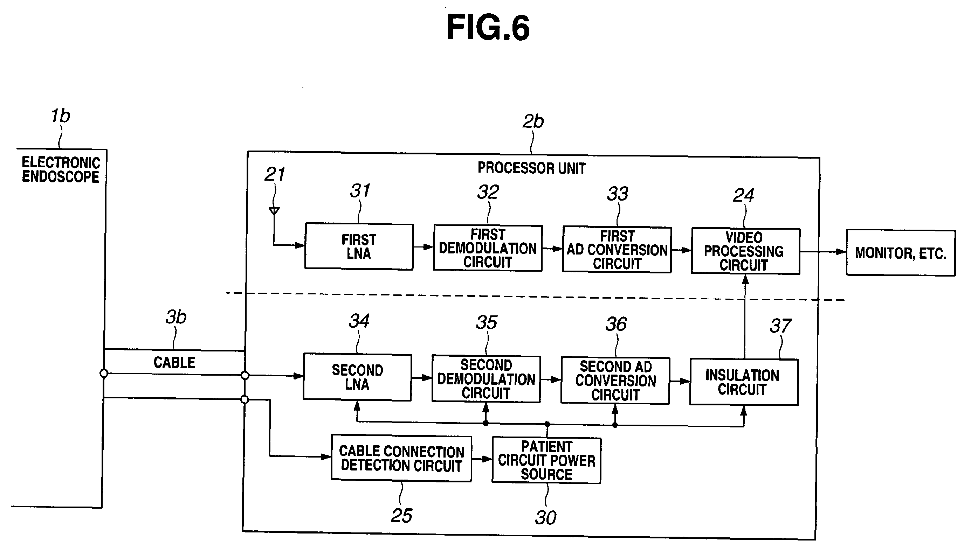

Fig. 6 is a block diagram illustrating a configuration for reducing power consumption of the processor unit. A processor unit 2b has a circuit for radio transmission, which includes a first LNA 31, a first demodulation circuit 32, a first AD conversion circuit 33 and the video processing circuit 24. The processor unit 2b also has the cable connection detection circuit 25. Also, the processor unit 2b has a patient circuit, which includes a patient circuit power source 30, a second LNA 34, a second demodulation circuit 35, a second AD conversion circuit 36 and an insulation circuit 37. The patient circuit power source 30 supplies power to the second LNA 34, the second demodulation circuit 35, the second AD conversion circuit 36 and the insulation circuit 37. When radio transmission is performed in the configuration shown in Fig. 6, the patient circuit power source 30 of the patient circuit out of use may be turned off based on the results of the detection derived from the cable connection detection circuit 25 provided at the processor unit 2b, so that the power consumption of the processor unit 2b can be reduced.

The present invention is not intended to be limited to the embodiments described above, but may be variously changed, modified, etc. within the spirit of the invention.

## Claims

1. An electronic endoscope apparatus comprising:
an electronic endoscope including a transmission circuit for performing radio transmission and cable transmission of an image pickup signal obtained by an image pickup element;
a processor unit including a reception circuit for receiving the image pickup signal transmitted by radio transmission and cable transmission, and for producing a video signal from the image pickup signal; and
gain changing means for changing gain of the transmission circuit and gain of the reception circuit, upon detecting connection of a cable between the electronic endoscope and the processor unit, the connection being for performing cable transmission of the image pickup signal.

2. The electronic endoscope apparatus according to claim 1, wherein:
the apparatus comprises cable connection detecting means for detecting connection of the cable; and
the gain changing means changes gain of the transmission circuit or gain of the reception circuit, upon detecting a detection signal indicative of a connection of the cable, from the cable connection detecting means.

3. The electronic endoscope apparatus according to claim 2, wherein:
the apparatus comprises switching means for performing switching between the radio transmission and the cable transmission to connect an output of the transmission circuit to an antenna or the cable; and
the switching means performs switching between the radio transmission and the cable transmission to establish connection with the antenna or the cable, on the basis of the detection signal from the cable connection detecting means.

4. The electronic endoscope apparatus according to any one of claims 1 to 3, wherein:
the antenna for performing the radio transmission between the electronic endoscope, and the processor unit is detachable and mounted in a cable connector for performing the cable transmission.

5. The electronic endoscope apparatus according to any one of claims 1 to 3, wherein:
the gain changing means changes gain of the transmission circuit or gain of the reception circuit to gain for performing the cable transmission, when a connection of the cable is detected.

6. The electronic endoscope apparatus according to any one of claims 1 to 3, wherein:
the gain changing means changes gain of the transmission circuit or gain of the reception circuit to gain for performing the radio transmission, when a connection of the cable is not detected.

7. The electronic endoscope apparatus according to claim 2 or 3, wherein:
the cable connection detecting means outputs the detection signal to the gain changing means on the basis of a connection of the cable.

8. The electronic endoscope apparatus according to any one of claims 1 to 3, wherein:
the transmission circuit is made up of a power amplifier.

9. The electronic endoscope apparatus according to any one of claims 1 to 3, wherein:
the reception circuit is made up of a low noise amplifier.

10. An electronic endoscope apparatus comprising:
an electronic endoscope having a transmission circuit for performing radio transmission and cable transmission of an image pickup signal obtained by an image pickup element;
a processor unit including a reception circuit for receiving the image pickup signal transmitted by radio transmission and cable transmission, and for producing a video signal from the image pickup signal; and
correcting means which is provided at a cable for performing cable transmission of the image pickup signal from the electronic endoscope to the processor unit, for correcting amplitude of the image pickup signal in the cable transmission.

11. The electronic endoscope apparatus according to claim 10, wherein:
the apparatus comprises:
cable connection detecting means for detecting connection of the cable; and
switching means for performing switching between the radio transmission and the cable transmission to connect an output of the transmission circuit to an antenna or the cable, and,
the switching means performs switching between the radio transmission and the cable transmission to establish connection with the antenna or the cable, on the basis of the detection signal from the cable connection detecting means.

12. The electronic endoscope apparatus according to claim 10 or 11, wherein:
the antenna for performing the radio transmission between the electronic endoscope and the processor unit is detachable, and mounted in a cable connector for performing the cable transmission.

13. The electronic endoscope apparatus according to claim 10 or 11, wherein:
the gain changing means changes gain of the transmission circuit or gain of the reception circuit to gain for performing the cable transmission, when a connection of the cable is detected.

14. The electronic endoscope apparatus according to claim 10 or 11, wherein:
the gain changing means changes gain of the transmission circuit or gain of the reception circuit to gain for performing the radio transmission, when a connection of the cable is not detected.

15. The electronic endoscope apparatus according to claim 11, wherein:
the cable connection detecting means outputs the detection signal to the gain changing means and the switching means on the basis of a connection of the cable.

16. The electronic endoscope apparatus according to claim 10 or 11, wherein:
the transmission circuit is made up of a power amplifier.

17. The electronic endoscope apparatus according to claim 10 or 11, wherein:
the reception circuit is made up of a low noise amplifier.

18. The electronic endoscope apparatus according to claim 10 or 11, wherein:
the correcting means is disposed in the vicinity of the electronic endoscope.

19. The electronic endoscope apparatus according to claim 10 or 11, wherein:
the correcting means is made up of an attenuator.

20. The electronic endoscope apparatus according to claim 10 or 11, wherein:
the correcting means is made up of a semiconductor circuit.
